# EUROPEAN PATENT SPECIFICATION

(11) **EP 2 123 315 B1**
(45) Date of publication and mention of the grant of the patent: **31.10.2012**
(21) Application number: 09160458.7
(22) Date of filing: 18.05.2009
(51) Int. Cl.: A61M 1/36

(54) **Device for collecting blood in an extracorporeal circuit comprising a venous blood reservoir and a cardiotomy reservoir**
Vorrichtung zum Sammeln von Blut in einem extrakorporalen Kreislauf mit einem Reservoir für venöses Blut und einem Kardiotomiereservoir
Dispositif de collecte de sang dans un circuit extracorporel comportant un réservoir sanguin veineux et un réservoir de cardiotomie

(30) Priority: 22.05.2008 IT MI20080946
(43) Date of publication of application: 25.11.2009
(73) Proprietor: Eurosets S.r.l., 41036 Medolla (MO) (IT)
(72) Inventor: Petralia, Antonio, 48022, Lugo RA (IT); Ghelli, Nicola, 40018, S. Pietro in Casale BO (IT); Costa Maianti, Edgardo, 41037, Mirandola MO (IT); Balanzoni, Roberto, 46020, San Giovanni Del Dosso MN (IT)
(74) Representative: Zoli, Filippo

(56) References cited:
- WO-A2-96/24397
- US-A- 5 158 533
- US-A- 6 017 493

## Description

The invention relates to device for collecting blood in an extracorporeal circuit which comprises a venous blood reservoir and a cardiotomy reservoir.

It is known that the extracorporeal blood circulation circuits that are activated during certain surgical procedures include, among others, a device that comprises two apparatuses shaped like interconnected containers, known respectively as "venous blood reservoir" and "cardiotomy reservoir".

The venous blood reservoir is provided with a coupling to a line for conveying the venous blood that arrives from the patient's heart and is aspirated by gravity or by way of a certain degree of vacuum that is created within the reservoir proper, and usually also with a coupling to a line for the conveyance of venous blood drawn from the aorta by means of a cannula known as "intracavitary vent", generally provided with a peristaltic pump; said reservoir is also provided with a connector for the outflow of blood toward the additional devices that are present in the extracorporeal circuit.

The cardiotomy reservoir is provided with a coupling to a line for the conveyance of the blood drawn from the operating field, and said conveyance is provided by means of a peristaltic pump.

As mentioned, the two devices are interconnected, but the methods for transferring blood from one apparatus to the other occur in ways that are not entirely satisfactory.

US 6,017,493 discloses a device as defined in the preamble of claim 1.

The aim of the present invention is therefore to provide a device of the described type in which the ways of transferring blood from one of the apparatuses that compose said device to the other are optimized, and which further allows to eliminate the pumps provided on the lines for conveying blood thereto.

This aim is achieved by a device for collecting blood in an extracorporeal circuit comprising a venous blood reservoir and a cardiotomy reservoir, characterized in that it comprises the features given in the appended claims.

Further characteristics and advantages will become better apparent from the description of a preferred but not exclusive embodiment of the device according to the invention, illustrated by way of non-limiting example in the accompanying drawings, wherein:
Figure 1 is a schematic view of the device according to the invention in normal operating conditions;
Figures 2, 3, 4 are views of the device in different situations of transfer of blood from one of the apparatuses comprised within the device to the other, as will become better apparent hereinafter.

With reference to the figures, the reference numeral 1 generally designates the device according to the invention, which comprises a venous blood reservoir 2 and a cardiotomy reservoir 3.

The venous blood reservoir 2, which comprises in a known manner within itself a filtering mass 2a, is provided with couplings 4 and 5, which are connected to lines that convey venous blood from a patient, drawing it respectively from the heart and from the aorta by means of the intracavitary vent, and is further provided with a connector 6 for the outflow of the blood toward additional apparatuses, such as an oxygenation device, comprised within the extracorporeal circuit.

The cardiotomy reservoir 3, which comprises in a known manner within itself a filtering mass 3 a, is provided with a coupling 7 connected to a line for conveying blood collected from the operating field.

The venous blood reservoir 2 and the cardiotomy reservoir 3 are connected to respective independent lines, designated respectively by the reference numerals 8 and 9, which lead to vacuum sources that are adapted to determine different degrees of vacuum in such apparatuses, the degree of vacuum related to the venous blood reservoir 2: merely by way of indication, as typical a value of the degree of vacuum equal to 0.13 bars in the cardiotomy reservoir and equal to 0.04 bars in the venous blood reservoir can be indicated, these values being such as to ensure respectively an effective removal of blood from the operating field without the aid of any pump, and a nontraumatic treatment of the venous blood drawn from the patient.

Line 9 in particular, and preferably also line 8, have a connection to the atmosphere, designated by the reference numeral 8a for the line 8 and 9a for the line 9, which is provided with removable blockage means, designated respectively by the reference numerals 8b and 9b, provided by means of a clamp-like apparatus that is known in the field as "clamp".

The device is provided with means for mutual connection of the venous blood reservoir and of the cardiotomy reservoir which comprise a first duct 10 and a second duct 11.

The first duct 10 is connected to said apparatuses substantially at the bottom: it in fact leads, at one end, to a connector 4a that extends from the bottom of the venous blood reservoir to accommodate the coupling 4, and is inserted, at the other end, in a valve 12 located at the bottom of the cardiotomy reservoir 3.

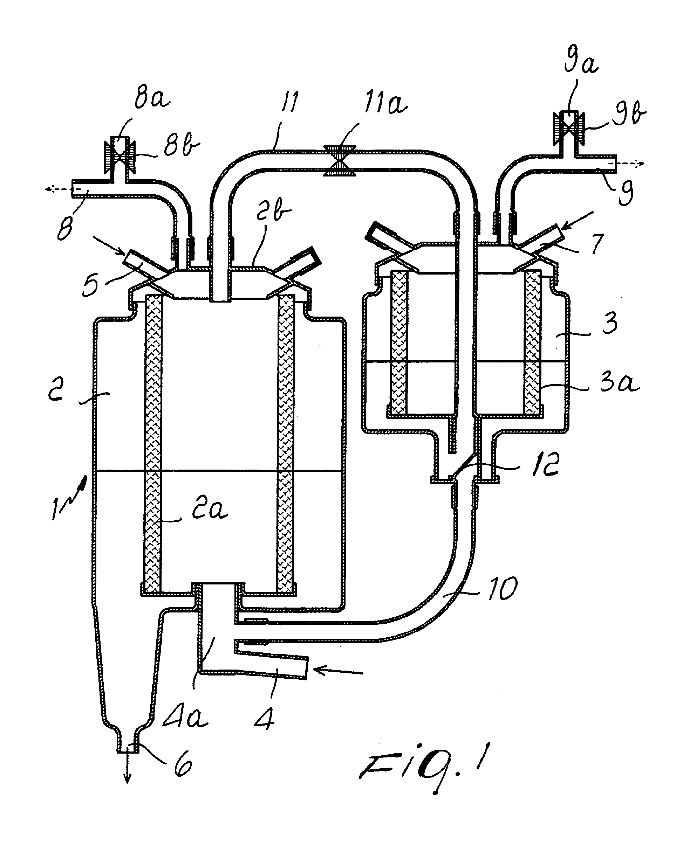

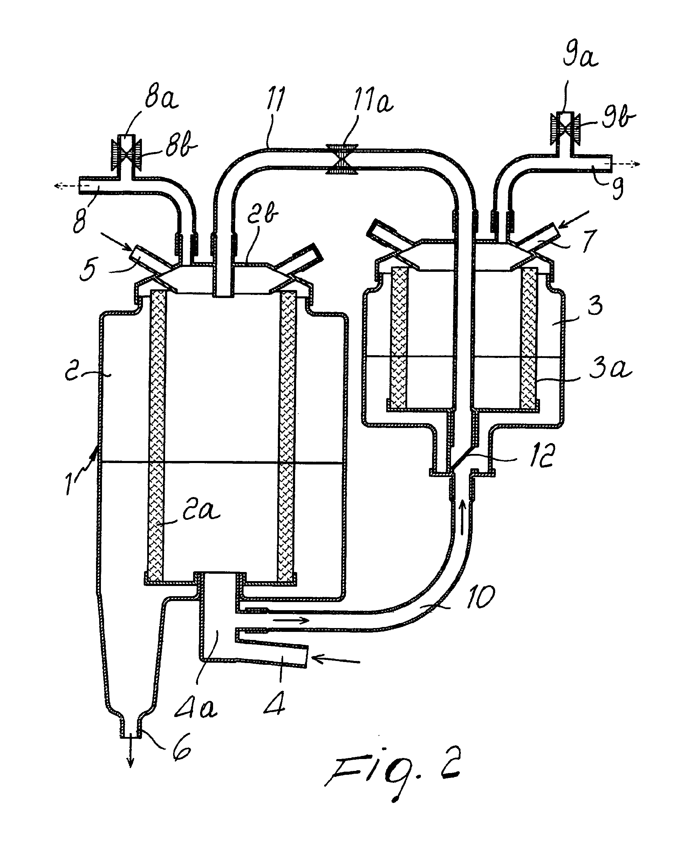

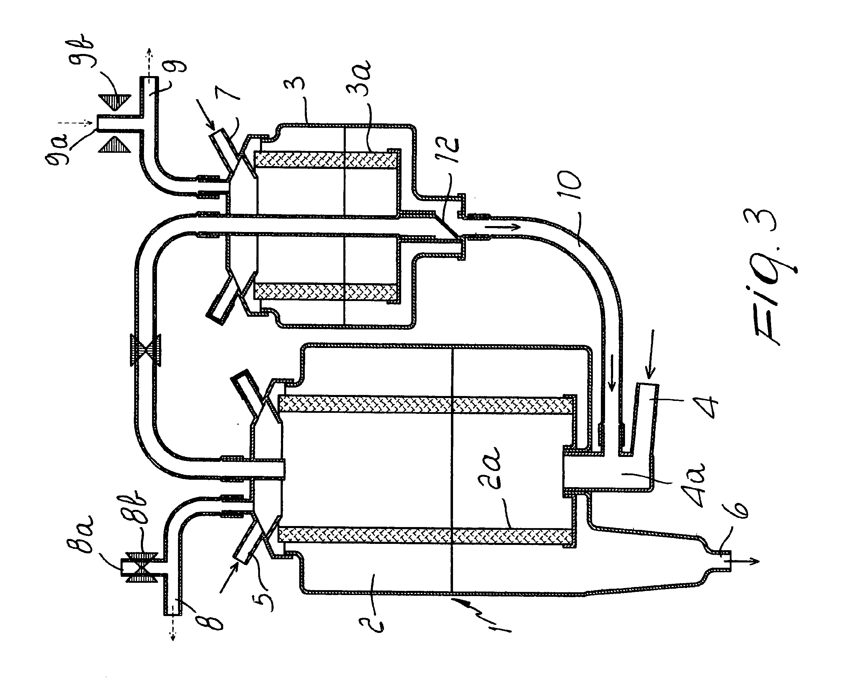

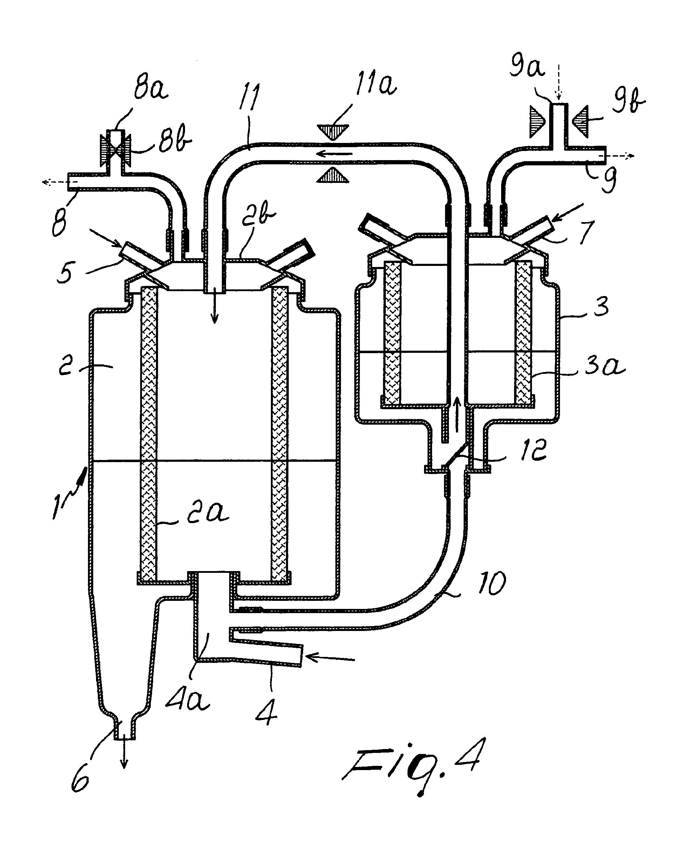

The second duct 11, provided with removable blockage means constituted by a clamp 11a, is connected at one end to an upper lid 2b of the venous blood reservoir 2 and leads out at the other end proximate to the bottom of the cardiotomy reservoir 3, and more precisely at the valve 12; the valve 12 can move between the position visible in Figures 1 and 4 for simultaneous blockage of the duct 10 and opening of the end of the duct 11, and the position visible in Figures 2 and 3 of simultaneous opening of the duct 10 and of blockage of the end of the duct 11.

On the lines connected to the couplings 5, 7 and to the connector 6 a one-way safety valve can be provided to prevent the occurrence of abnormal vacuum values within the two apparatuses.

In normal operating conditions, shown in Figure 1, the clamp 11a is closed and the valve 12 is in a position for blocking the duct 10; the venous blood arrives from the patient to the venous blood reservoir 2 by means of the couplings 4 and 5, and from the operating field to the cardiotomy reservoir 3 by means of the coupling 7, all as indicated by the arrows in the figure, thanks to the appropriate degrees of vacuum that are present in said apparatuses.

If the need arises to transfer blood from the venous reservoir 2 to the cardiotomy reservoir 3, it is sufficient to move the valve 12 to the position of Figure 2, leaving the clamp 11a closed: the blood, following the arrows in the figure, passes from the venous reservoir 2 to the cardiotomy reservoir 3, flowing along the duct 10, attracted by the higher degree of vacuum.

If vice versa it is necessary to transfer blood from the cardiotomy reservoir 3 to the venous reservoir 2, it is necessary to perform a first operation, which consists in opening the clamp 9b, obtaining the effect of bringing the cardiotomy reservoir 3 to atmospheric pressure, and at this point two possibilities arise.

It is in fact possible to work as indicated in Figure 3: leaving the clamp 11a closed, the valve 12 is moved to the open position of the duct 10 and thus the blood passes from the cardiotomy reservoir 3 at atmospheric pressure to the reservoir 2, in which there is a certain degree of vacuum, following the duct 10 as shown by the arrows in the figure.

However, the operating possibility shown in Figure 4 is also offered, in which the clamp 11a is opened and the valve 12 is moved to the position for blocking the duct 10: thanks to the fact that the blood is drawn from the cardiotomy reservoir 3 at atmospheric pressure at the bottom, the blood passes into the venous blood reservoir 2, which is in vacuum, following the duct 11 along the arrows shown in the figure until the cardiotomy reservoir is emptied completely.

This operating condition is particularly advantageous: if the clamp 11a is in fact left open after the cardiotomy reservoir has achieved complete emptying, air reaches the reservoir 2, but its entry from above excludes any possibility of mixing with the blood, which therefore remains entirely free from traumas.

The described invention is susceptible of numerous modifications and variations, as far as these are within the scope of the appended claims; all the details may further be replaced with other technically equivalent elements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device (1) for collecting blood in an extracorporeal circuit comprising a venous blood reservoir (2) and a cardiotomy reservoir (3), said venous blood reservoir (2) and said cardiotomy reservoir (3) being interconnected and being provided with couplings (4, 5) respectively to at least one line for conveying venous blood from the patient and to a line for conveying blood collected from the operating field, said venous blood reservoir (2) being further provided with a connector (6) for the outflow of the blood toward additional apparatuses comprised within said extracorporeal circuit, said venous blood reservoir (2) and said cardiotomy reservoir (3) being connected to respective independent lines (8, 9) that reach vacuum sources and having, at least as regards the line (9) related to the cardiotomy reservoir (3), a connection (9a) to the atmosphere provided with removable blocking means (9b), there being also means for the mutual connection of the venous blood reservoir (2) and the cardiotomy reservoir (3) which comprise a first duct (10) that is connected at its ends to said apparatuses at the bottom, **characterized in that** said vacuum sources are adapted to determine different degrees of vacuum in said apparatuses, the degree of vacuum related to the cardiotomy reservoir (3) being higher than the degree of vacuum related to the venous blood reservoir (2), said first duct (10) being provided with removable blocking means (12) and there being a second duct (11), provided with removable blocking means (11a), which is connected at one end to the upper lid (2b) of the venous blood reservoir (2) and leads at the other end proximate to the bottom of the cardiotomy reservoir (3).

2. The device according to claim 1, **characterized in that** the first duct (10) is provided with removable blocking means (12), which comprise a valve (12) located at the end that is connected to the bottom of the cardiotomy reservoir (3).

3. The device according to claim 1, **characterized in that** the first duct (10) is provided with removable blockage means (12), which comprise a valve (12) located at the end that is connected to the bottom of the cardiotomy reservoir (3), which can move between a position for simultaneous blocking of said first duct (10) and opening of the end of the second duct (11) that leads into the cardiotomy reservoir (3), and a position for simultaneous opening of said first duct (10) and for blocking said end of the second duct (11).

4. The device according to one or more of the preceding claims, **characterized in that** the removable means (11a) for blocking the second duct (11) comprise a clamp (11a).

5. The device according to one or more of the preceding claims, comprising a venous blood reservoir (2) which has, in addition to a first coupling (4, 5, 7) to a line for conveying venous blood from the heart of a patient, a second coupling to a line for conveying blood drawn by means of an intracavitary vent, **characterized by** the presence of a one-way and safety valve on the lines connected respectively to said second coupling, to the coupling (7) provided on the cardiotomy reservoir (3) and to the output connector (6) of the venous blood reservoir (2).

## Patentansprüche

1. Vorrichtung (1) zum Sammeln von Blut in einem extrakorporalen Kreislauf mit einem Reservoir (2) für venöses Blut und einem Kardiotomiereservoir (3), wobei das Reservoir für venöses Blut (2) und das Kardiotomiereservoir (3) miteinander verbunden und mit Kupplungen (4, 5) zu mindestens einer Leitung zum Befördern von venösem Blut vom Patienten bzw. zu einer Leitung zum Befördern von aus dem Operationsfeld gesammeltem Blut versehen sind, wobei das Reservoir (2) für venöses Blut ferner mit einem Anschluss (6) für das Ausfließen des Blutes in Richtung zusätzlicher, innerhalb des extrakorporalen Kreislaufs enthaltener Apparate versehen ist, wobei das Reservoir (2) für venöses Blut und das Kardiotomierservoir (3) mit jeweiligen unabhängigen Leitungen (8, 9), die Vakuumquellen erreichen, verbunden sind und, zumindest was die Leitung (9) bezüglich des Kardiotomiereservoirs (3) betrifft, eine mit entfernbaren Sperreinrichtungen (9b) versehene Verbindung (9a) zur Atmosphäre besitzen, wobei außerdem Einrichtungen zum wechselseitigen Verbinden des Reservoirs (2) für venöses Blut und des Kardiotomiereservoirs (3) vorhanden sind, die einen ersten Kanal (10), der an seinen Enden mit den Apparaten an der Unterseite verbunden ist, aufweisen, **dadurch gekennzeichnet, dass** die Vakuumquellen angepasst sind, unterschiedliche Vakuumgrade in den Apparaten zu bestimmen, wobei der Vakuumgrad bezüglich des Kardiotomiereservoirs (3) höher als der Vakuumgrad bezüglich des Reservoirs (8) für venöses Blut ist, wobei der erste Kanal (10) mit entfernbaren Sperreinrichtungen (12) versehen ist und wobei ein mit entfernbaren Sperreinrichtungen (11a) versehener, zweiter Kanal (11) vorhanden ist, der an einem Ende mit dem oberen Deckel (2b) des Reservoirs (2) für venöses Blut verbunden ist und am anderen Ende in die Nähe der Unterseite des Kardiotomiereservoirs (3) führt.

2. Vorrichtung nach Anspruch 1, **dadurch gekennzeichnet, dass** der erste Kanal (10) mit einer entfernbaren Sperreinrichtung (12) versehen ist, die ein Ventil (12), das sich an dem Ende befindet, das mit der Unterseite des Kardiotomiereservoirs (3) verbunden ist, aufweist.

3. Vorrichtung nach Anspruch 1, **dadurch gekennzeichnet, dass** der erste Kanal (10) mit einer entfernbaren Sperreinrichtung (12) versehen ist, die ein Ventil (12), das sich an dem Ende befindet, das mit der Unterseite des Kardiotomiereservoirs (3) verbunden ist, aufweist, die sich zwischen einer Stellung für das gleichzeitige Sperren des ersten Kanals (10) und Öffnen des Endes des zweiten Kanals (11), der in das Kardiotomiereservoir (3) führt, und einer Stellung für das gleichzeitige Öffnen des ersten Kanals (10) und für das Sperren des Endes des zweiten Kanals (11) bewegen kann.

4. Vorrichtung nach einem oder mehr der vorangegangenen Ansprüche, **dadurch gekennzeichnet, dass** die entfernbare Einrichtung (11a) zum Sperren des zweiten Kanals (11) eine Klemme (11a) aufweist.

5. Vorrichtung nach einem oder mehr der vorangegangenen Ansprüche, mit einem Reservoir (2) für venöses Blut, das zusätzlich zu einer ersten Kupplung (4, 5, 7) zu einer Leitung zum Befördern von venösem Blut aus dem Herzen eines Patienten eine zweite Kupplung zu einer Leitung zum Befördern von mittels einer intrakavitären Abzugsöffnung abgezogenem Blut besitzt, **gekennzeichnet durch** das Vorhandensein eines Einwege- und Sicherheitsventils an den Leitungen, die mit der zweiten Kupplung, mit der an dem Kardiotomiereservoir (3) bereitgestellten Kupplung (7) bzw. mit dem Ausgangsanschluss (6) des Reservoirs (2) für venöses Blut verbunden sind.

## Revendications

1. Dispositif (1) pour recueillir du sang dans un circuit extracorporel comprenant un réservoir (2) pour du sang veineux et un réservoir de cardiotomie (3), ledit réservoir (2) pour du sang veineux et ledit réservoir de cardiotomie (3) étant interconnectés et étant pourvus de manchons (4, 5) d'accouplement respectivement à au moins une ligne d'acheminement du sang veineux provenant du patient et à une ligne pour acheminer le sang collecté du champ opératoire, ledit réservoir (2) de sang veineux étant en outre doté d'un raccord (6) pour l'écoulement du sang vers des dispositifs supplémentaires présents dans ledit circuit extracorporel, ledit réservoir (2) de sang veineux et ledit réservoir de cardiotomie (3) étant reliés à des lignes indépendantes respectives (8, 9) qui mènent à des sources de vide et ayant, au moins en ce qui concerne la ligne (9) reliée au réservoir de cardiotomie (3), une liaison (9a) avec l'atmosphère, dotée de moyens d'obturation amovibles (9b), étant également présents des moyens pour la connexion entre eux du réservoir (2) de sang veineux et du réservoir de cardiotomie (3) qui comprennent un premier conduit (10) qui est raccordé à ses extrémités auxdits dispositifs en partie inférieure, ***caractérisé en ce que*** lesdites sources de vide sont aptes à déterminer différents degrés de vide dans lesdits dispositifs, le degré de vide concernant le réservoir de cardiotomie (3) étant supérieur au degré de vide concernant le réservoir (2) de sang veineux, ledit premier conduit (10) étant doté de moyens d'obturation amovibles (12), et un deuxième conduit (11) étant présent, doté de moyens d'obturation amovibles (11a), deuxième conduit qui est raccordé à une extrémité du couvercle supérieur (2b) du réservoir (2) de sang veineux et amène, à l'autre extrémité, à proximité du fond du réservoir de cardiotomie (3).

2. Dispositif selon la revendication 1, ***caractérisé en ce que*** le premier conduit (10) est doté de moyens d'obturation amovibles (12) qui comprennent une valve (12) située à l'extrémité qui est raccordée au fond du réservoir de cardiotomie (3).

3. Dispositif selon la revendication 1, ***caractérisé en ce que*** le premier conduit (10) est doté de moyens d'obturation amovibles (12) qui comprennent une valve (12) située à l'extrémité qui est raccordée au fond du réservoir de cardiotomie (3), qui peut être déplacée entre une position pour simultanément obturer ledit premier conduit (10) et ouvrir l'extrémité du deuxième conduit (11) qui aboutit dans le réservoir de cardiotomie (3), et une position pour simultanément ouvrir ledit premier conduit (10) et obturer ladite extrémité du deuxième conduit (11).

4. Dispositif selon l'une ou plusieurs des revendications précédentes, ***caractérisé en ce que*** les moyens amovibles (11a) pour bloquer le deuxième conduit (11) comprennent un clamp (11a).

5. Dispositif selon l'une ou plusieurs des revendications précédentes, comprenant un réservoir (2) de sang veineux qui possède, outre un premier manchon (4, 5, 7) d'accouplement à une ligne d'acheminement du sang veineux provenant du coeur d'un patient, un deuxième manchon d'accouplement à une ligne d'acheminement du sang soutiré au moyen d'une canule intracavitaire, ***caractérisé par*** la présence d'une valve unidirectionnelle et de sécurité sur les lignes raccordées respectivement audit deuxième manchon d'accouplement, au manchon d'accouplement (7) présent sur le réservoir de cardiotomie (3) et au raccord (6) de sortie du réservoir (2) de sang veineux.
